# EUROPÄISCHE PATENTSCHRIFT

(11) **EP 1 017 696 B1**
(45) Veröffentlichungstag und Bekanntmachung des Hinweises auf die Patenterteilung: **20.03.2002**
(21) Anmeldenummer: 98945312.1
(22) Anmeldetag: 05.09.1998
(51) Int. Cl.: C07D 489/04, A61K 31/485

(54) **SÄUREADDITIONSSALZE VON MORPHIN-ALKALOIDEN UND DEREN VERWENDUNG**
ACIDIC ADDITION SALTS OF MORPHINE ALKALOIDS AND THE APPLICATION THEREOF
SELS D'ADDITION D'ACIDE D'ALCALOIDES DE LA MORPHINE ET LEUR UTILISATION

(30) Priorität: 25.09.1997 DE 19742296; 29.07.1998 DE 19834005
(43) Veröffentlichungstag der Anmeldung: 12.07.2000
(73) Patentinhaber: LTS Lohmann Therapie-Systeme AG, 56626 Andernach (DE)
(72) Erfinder: HOFFMANN, Rainer, D-56566 Neuwied (DE); ASMUSSEN, Bodo, D-56170 Bendorf (DE); KOCH, Andreas, D-56581 Melsbach (DE); HILLE, Thomas, D-56567 Neuwied (DE); ADAM, Bernd, D-34613 Treysa (DE); MATUSCH, Rudolf, D-35041 Marburg (DE)
(74) Vertreter: Flaccus, Rolf-Dieter, Dr.
(86) Internationale Anmeldenummer: EP9805652
(87) Internationale Veröffentlichungsnummer: WO9915528

(56) Entgegenhaltungen:
- EP-A- 0 472 501
- WO-A-95/04058
- DE-A- 19 607 395
- DE-B- 1 172 267
- DE-C- 524 639
- DE-C- 728 804
- FR-A- 1 479 209
- FR-M- 6 598
- US-A- 4 626 539
- US-A- 4 879 297
- US-A- 4 908 389
- US-A- 5 374 645

## Beschreibung

Die Erfindung betrifft Stoffe, im wesentlichen bestehend aus dem Säureadditionssalz eines Morphin-Alkaloids und einer organischen Säure, wobei das Morphin-Alkaloid die nachfolgende Formel I aufweist: 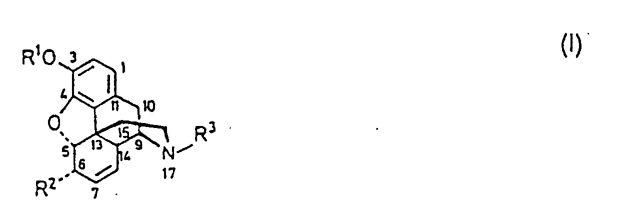 wobei R¹ ausgewählt ist aus der Gruppe, bestehend aus H, C₁- bis C₆-Alkylresten, bevorzugt Methyl-, Ethyl-, Propyl-, i-Propyl, C(O)CH_{3,} R² ausgewählt ist aus der Gruppe, bestehend aus H, OH, OC(O)CH₃, =O,=CH₂, R³ ausgewählt ist aus der Gruppe, bestehend aus -CH₃, Cyclopropyl-, Cyclobutyl- und Allyl-, sowie die Bindung an C7/C8 gesättigt sein oder am N₁₇ eine Nitroxylgruppe vorliegen kann.

Morphin-Alkaloide, insbesondere Morphin, gehören zu der Gruppe der starken Analgetika; ihr therapeutischer Einsatz liegt u. a. im Bereich der Behandlung stärker und stärkster Schmerzzustände, wie sie z. B. bei vielen Krebserkrankungen im Finalstadium oder auch nach Unfällen vorkommen.

Die bisherigen Applikationsmöglichkeiten (oral, parenteral) mit diesen Stoffen sind unbefriedigend. Dabei besteht die Gefahr von säurekatalysierten chemischen Veränderungen im Magen. Außerdem kommt es zu starken Schwankungen des Plasmaspiegels; dies wird insbesondere bei parenteraler Applikation (Injektion) beobachtet. Infolgedessen kommt es wegen Über- oder Unterschreitung der therapeutisch erwünschten Plasmakonzentrationen zu suchterzeugenden Effekten.

Aus der US-A 4,626,539 sind pharmazeutische Zusammensetzungen bekannt, die ein Opioid, wie beispielsweise Morphin, oder deren pharmazeutisch akzeptable Salze enthalten. Als pharmazeutisch akzeptable Salze sind in dieser Patentschrift Acetate, Napsylate, Tosylate, Succinate, Hydrochloride, Palmitate, Stearate, Oleate, Parmoate, Laurate, Valerate, Hydrobromide, Sulfate, Methansulfonate, Tartrate, Citrate und Maleate beschrieben.

Aus der US-A 5,374,645 sind Zusammensetzungen für die transdermale Verabreichung ionischer pharmazeutisch aktiver Agenzien bekannt, wobei zu den dabei erwähnten Stoffen auch Morphin oder dessen pharmazeutisch akzeptable Salze gehören. Als Salze werden zusätzlich zu den vorstehend erwähnten Morphinsalzen Oxalate, Pyruvate, Cinnamate, Acetate, Trifluoracetate sowie Salicylate und einige andere erwähnt.

Die US-A 4,879, 297 beschreibt pharmazeutische Zusammensetzungen, die Opioide oder deren pharmazeutisch akzeptable Salze enthalten, wobei als Salze insbesondere Salze von bestimmten Fettsäuren wie Palmitate, Stearate, Oleate, Parmoate beschrieben sind.

Des weiteren sind in der US-A 4,908,389 wirkstoffhaltige Zusammensetzungen zur topischen Applikation beschrieben, die die Wirkstoffe in Form der Säureadditionssalze wie der Hydrochloride, Hydrobromide, Orthophosphate, Benzoate, Maleate, Tartrate, Succinate, Citrate, Salicylate, Sulfate oder Acetate enthalten.

Bei der dermalen oder topischen Applikation eines dieser vorstehend genannten Säureadditionssalze von Morphin-Alkaloiden tritt der Nachteil auf, daß die Permeabilität der genannten Salze durch die Haut sehr gering ist. Dieser Nachteil wird versuchsweise bei den bekannten Zusammensetzungen dadurch kompensiert, daß man den Darreichungsformen sog. Enhancer zusetzt.

DE 524 639 C beschreibt Verfahren zur Darstellung von Sulfonsäuresalzen des Benzylmorphins, wobei Sulfonsäuren aliphatischer Kohlenwasserstoffe mit weniger als fünf C-Atomen zur Salzbildung verwendet werden. Die so erhaltenen Salze des Benzylmorphins sind leicht wasserlöslich; hingegen ist über deren Permeabilität durch die Haut nichts bekannt.

Auch wenn dies zu einem Teil den gewünschten Erfolg zeitigt, so ist es unter pharmazeutischen oder therapeutischen sowie zulassungsrechtlichen Gesichtspunkten bevorzugt, wenn Morphin-Alkaloidsaize bereitstünden, die per se eine höhere Permeabilität durch die Haut aufweisen, so daß keine weitere oder nur wenig einer weiteren Substanz erforderlich ist. Dies insbesondere deshalb, da die genannten Enhancer auch bei ihrer Verwendung auf der Haut zu Nachteilen wie etwa Hautreizungen oder unerwünschten pharmakodynamischen Nebenwirkungen infolge zu großer Toxizität führen können.

Aufgabe der Erfindung ist es deshalb, Säureadditionssalze von Morphin-Alkaloiden der vorstehend genannten Formel I bereitzustellen, die gegenüber den bekannten Salzen verbesserte Eigenschaften aufweisen. Insbesondere soll ihre Permeabilität durch die Haut erhöht sein.

Diese Aufgabe wird dadurch gelöst, daß Säureadditionssalze eines Morphinalkaloids und einer organischen Säure bereitgestellt werden, wobei das Morphin-Alkaloid die nachfolgende Formel I aufweist: 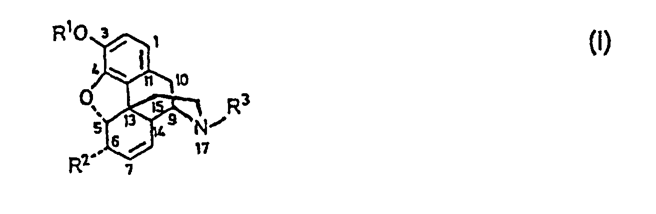 wobei R¹ ausgewählt ist aus der Gruppe, bestehend aus H, C₁- bis C₆-Alkylresten, bevorzugt Methyl-, Ethyl-, Propyl-, i-Propyl, C(O)CH₃, R² ausgewählt ist aus der Gruppe, bestehend aus den einwertigen Resten H, OH, OC(O)CH₃, wobei in diesem Falle die vierte Valenz des (6)-C-Atoms durch H belegt ist, oder den zweiwertigen Resten =O, =CH₂, R³ ausgewählt ist aus der Gruppe, bestehend aus -CH₃, Cyclopropyl-, Cyclobutyl- und Allyl- sowie
- die Bindung an C7/C8 gesättigt sein oder am N₁₇ eine Nitroxylgruppe vorliegen kann,
wobei die organische Säure ausgewählt ist aus:
- Monoestern von C₃- bis C₁₆-Dicarbonsäuren mit einwertigen C₁- bis C₄-Alkoholen, insbesondere Methanol,
- C₂- bis C₆- oder C₈- bis C₁₆-Sulfonsäuren,
- der Gruppe der halogen-, *p*- und *m*-hydroxy-, alkyl-, hydroxyalkyl-, alkoxyalkyl-, und/oder alkoxysubstituierten Benzoesäuren sowie der wahlweise am N-Atom alkylierten aminosubstituierten Benzoesäuren,
- substituierten oder nicht substituierten 5- oder 6-Ringheterocyclen mit mindestens einem N-Atom oder S-Atom und mit einer Carboxylgruppenfunktion, insbesondere einer Carboxy-, carboxymethyl-, Carboxyethyl- oder den wahlweise verzweigten Carboxypropyl- oder Carboxybutylgruppen als Substituenten,
- gesättigten oder ungesättigten, wahlweise substituierten Oxo-Carbonsäureen mit 5 bis 10 C-Atomen,
- phenoxysubstituierten gesättigten C₂- bis C₄-Carbonsäuren,
- aliphatischen, aromatischen oder heterocyclischen C₂-C₁₂-Aminosäuren, worin eine Aminogruppe substituiert ist mit einer wahlweise substituierten C₂-C₆-Alkanoylgruppe oder einer wahlweise substituierten Benzoylgruppe, und
- und wobei das Säureadditionssalz eine Hautpermeabilität mit einem Flux von mindestens 2,34 µg/cm²·h besitzt.

Bevorzugte Ausführungsformen sind Gegenstand der Unteransprüche.

Die Erfindung umfaßt somit Stoffe, die im wesentlichen aus dem Säureadditionssalz eines Morphin-Alkaloids der vorstehend genannten Formel I und einer weiteren organischen Säure. Der Ausdruck "im wesentlichen bestehend aus" bedeutet, daß Verunreinigungen nur im üblichen Umfang enthalten sind. Der erfindungsgemäße Stoff bzw. die erfindungsgemäße Substanz kann mittels üblicher Verfahren der präparativen organischen Chemie hergestellt und aufgereinigt werden, so daß der gereinigte Stoff auch in p.A.- oder p.p.A.-Reinheit zur Verfügung gestellt werden kann. Die Säure ist insbesondere pharmazeutisch akzeptabel. Auch sie kann mittels üblicher Verfahren hergestellt werden, sofern sie nicht bereits käuflich erworben werden kann.
Bei dem Morphin-Alkaloid der vorstehend genannten Formel l ist R¹ ausgewählt aus der Gruppe, bestehend aus H, C₁-bis C₆-Alkylresten, C(O)CH₃. Bei den C₁-bis C₆-Alkylresten handelt es sich bevorzugt um Methyl-, Ethyl-, Propyl- oder i-Propylreste. Bei dem Rest R² handelt es sich um einen einwertigen Rest aus der Gruppe H, OH, OC(O)CH₃, wobei dann die vierte Valenz am (6)-C-Atom durch H belegt ist. Alternativ dazu kann R² auch einer der zweiwertigen Reste =O oder =CH₂ sein. R³ ist ausgewählt aus der Gruppe, bestehend aus -CH₃, Cyclopropyl-, Cyclobutyl- und Allyl-. Des weiteren kann die Doppelbindung zwischen C7/C8 gesättigt sein. Außerdem kann am N 17 eine Nitroxylgruppe vorliegen. Bei den vorstehend aufgezählten organischen Resten steht C(O) für eine Carbonylfunktion.

Die Säurekomponente des erfindungsgemäßen Säureadditionssalzes ist ausgewählt aus Monoestern von C₃- bis C₁₆-Dicarbonsäuren mit einwertigen C₁- bis C₄-Alkoholen, aus C₂- bis C₆- oder C₈- bis C₁₆-Sulfonsäuren, aus der Gruppe der halogen-, *p*- und *m*-hydroxy-, alkyl-, hydroxyalkyl-, alkoxyalkyl-, und /oder alkoxysubstituierten Benzoesäuren sowie der wahlweise am N-Atom alkylierten aminosubstituierten Benzoesäuren ausgewählt sind, aus substituierten oder nichtsubstituierten, gesättigten oder nicht-gesättigten 5- oder 6-Ringheterocyclen mit mindestens einem N-Atom oder S-Atom und mit einer der bereits erwähnten Carboxylgruppenfunktionen als Substituenten, ganz besonders bevorzugt einer Carboxylgruppe als Substituenten, aus gesättigten oder ungesättigten, wahlweise substituierten Oxo-Carbonsäuren mit 5 bis 10 C-Atomen oder aus phenyl- oder phenoxysubstituierten gesättigten C₂- bis C₄-Carbonsäuren, insbesondere Essigsäure. Unter C₃- bis C₁₆-Dicarbonsäuren werden hier natürlich Carbonsäuren mit einer Gesamtkohlenstoffzahl von 5 bis 18 C-Atomen verstanden.

Bei den alkyl-, hydroxyalkyl- oder alkoxyalkylsubstituierten Benzoesäuren, handelt es sich insbesondere um solche, bei denen der Alkylrest oder auch der Alkoxyrest am Benzoesäurekern 1 bis 12 C-Atome aufweist. Diese Alkyl- bzw. Alkoxyreste können auch verzweigt sein. Beispiele dafür sind etwa i-Propyl-, 2-Methylpropyl, t-Butylreste, 2-Methylbutylreste bzw. die entsprechenden Alkoxyreste. Die Benzoesäurekeme können auch mehrfach, natürlich auch mit verschiedenen der erwähnten Alkyl- bzw. Alkoxyreste substituiert sein.

Bei mit Alkoxyalkylresten substituierten Benzoesäuren gilt bzgl. des Alkyl- oder Alkoxyteils des Alkoxyalkylrestes in bezug auf die Kohlenstoffatomzahl oder die Verzweigung das gleiche, wie vorstehend zu den Alkyl- bzw. Alkoxyresten am Benzoesäurekern der alkyl- oder alkoxysubstituierten Benzoesäuren ausgeführt.

Als Alkoxy-Substituenten in bevorzugten alkoxyalkylsubstituierten Benzoesäuren kommen ebenfalls bevorzugt C₁-bis C₆-Alkoxygruppen, insbesondere Methyloxy-, Ethyloxy- oder Propyloxy-Gruppen in Betracht. Diese Alkoxygruppen sind mit C₁- bis C₄-Hydroxyalkyl-, insbesondere mit Hydroxymethyl-, Hydroxyethyl- oder Hydroxypropylgruppen verethert.

Die genannten aminosubstituierten Benzoesäuren können wahlweise an der Aminogruppe ebenfalls - insbesondere mit C₁- bis C₄-Alkylresten- alkyliert sein.

Bei den substituierten Benzoesäuren handelt es sich bevorzugt um halogen-, C₁- bis C₆-alkyl-, hydroxy-(C₁- bis C₆)-alkyl-, aminosubstituierte oder hydroxysubstituierte Benzoesäuren. Die aminosubstituierten Benzoesäuren können ihrerseits wieder an der Aminogruppe substituiert sein - wie bereits vorstehend erwähnt. Handelt es sich um Aminobenzoesäure, so ist die Aminogruppe bei bevorzugten Ausführungsformen entweder unsubstituiert oder mit C₁-bis C₄-Alkylgruppen mono- oder disubstituiert. Als besonders bevorzugte alkylsubstituierte Benzoesäuren kommen ein- oder mehrfach C₁- bis C₄ alkylsubstituierte Benzoesäuren, bevorzugt C₁-bis C₄-trialkylsubstituierte Benzoesäuren in Frage, wobei die Alkylreste auch verschieden sein können.

Beispiele für bevorzugte hydroxyalkylsubstituierte Benzoesäuren sind hydroxymethylierte, -ethylierte-, -propylierte- oder -butylierte Benzoesäuren.

Von den vorstehend erwähnten hydroxysubstituierten Benzoesäuren sind ganz besonders die p- oder m-hydroxysubstituierten Benzoesäuren bevorzugt.

Am allerbevorzugtesten unter den substituierten Benzoesäuren für die Säurekomponente der erfindungsgemäßen Säureadditionssalze von Morphin-Alkaloiden der vorstehend genannten Formel I sind p-Hydroxybenzoesäure, p-Aminobenzoesäure oder Trimethylbenzoesäure, insbesondere 2,4,6-Trimethylbenzoesäure.

Bei den erfindungsgemäß als Säurekomponente für die Morphin-Alkaloid-Säureadditionssalze eingesetzten substituierten oder nichtsubstituierten 5- oder 6-Ringheterocyclen handelt es sich um cyclische 5- oder 6-Ringsysteme, die mindestens ein Stickstoff- oder S-Atom aufweisen, wie insbesondere Pyridin-, Piperidin-, Pyrimidin- oder analoge Pyrrol- oder Thiophen-Ringsysteme. Diese Ringsysteme tragen außerdem an einem Ringatom eine Carboxylgruppe. Natürlich kann das heterocyclische Ringsystem auch gesättigt sein, wie bereits anhand des Piperidinringsystems deutlich wird.

Bevorzugt handelt es sich bei den 6-Ringheterocyclen um eine substituierte oder nicht-substituierte Pyridincarbonsäure, insbesondere Nicotinsäure. Zu den bevorzugten 5-Ringsystemen mit mindestens einem S-Atom gehört Liponsäure.

Die erfindungsgemäßen Morphin-Alkaloid-Säureadditionssalze können - wie bereits erwähnt - in bezug auf die Säurekomponente auch aus C₂-bis C₁₆-Sulfonsäuren bestehen. Unter diesen Sulfonsäuren sind C₄-bis C₈-Sulfonsäuren, insbesondere Hexansulfonsäure, bevorzugt.

Die bei den erfindungsgemäßen Morphin-Alkaloid-Säureadditionssalzen eingesetzten Monoester von C₃-bis C₁₆-Dicarbonsäuren mit einwertigen C₁- bis C₄-Alkoholen, insbesondere Methanol, sind bevorzugt Monoester von C₅-bis C₁₀-Dicarbonsäuren mit den vorstehend genannten Alkoholen.

Besonders bevorzugt sind dabei als Säuren Suberinsäuren, Azelainsäure oder Sebacinsäure. Am allerbevorzugtesten ist unter den vorstehend genannten Monoestem von Dicarbonsäuren Monomethylsebacat.

Wird erfindungsgemäß eine gesättigte oder ungesättigte, z. B. olefinisch ungesättigte, wahlweise substituierte Oxo-Carbonsäure mit 5 bis 10 C-Atomen als Säurekomponente der Morphin-Alkaloid-Säureadditionssalze eingesetzt, so handelt es sich dabei bevorzugterweise um eine wahlweise olefinisch ungesättigte 2-, 4-, 5- oder 9-Oxo-Carbonsäure. Unter diesen Oxo-Carbonsäuren sind 5-Oxo-Pyrrolidin-2-Carbonsäure (Pyroglutaminsäure), Lävulinsäure oder Oxo-dec-2-ensäure am vorteilhaftesten.

Wird als Säurekomponente für die erfindungsgemäßen Morphin-Alkaloid-Säureadditionssalze eine phenyl- oder phenoxysubstituierte gesättigte C₂-bis C₄-Carbonsäure eingesetzt, so handelt es sich dabei vorzugsweise um eine phenyl- oder phenoxysubstituierte Essig-, Propion- oder Buttersäure.

Bei den erfindungsgemäß eingesetzten aliphatischen, aromatischen oder heterocyclischen C₂- C₁₂- Aminosäuren handelt es sich bevorzugt um Monoaminomonocarbonsäuren, worin die Aminogruppe substituiert ist mit einer C₂- C₆- Alkanoylgruppe, welche einfach oder mehrfach substituiert sein kann mit Hydroxy, C₁- C₄- Alkoxy- oder C₁- C₄- Hydroxyalkyl, oder worin die Aminogruppe substituiert ist mit dem Benzoylrest, welcher einfach oder mehrfach substituiert sein kann mit C₁-C₄- Alkyl, C₁- C₄- Alkoxy, C₁- C₄-Hydroxyalkyl, Halogen, Amino oder Hydroxy.

Bei den aromatischen Aminosäuren kann es sich z. B. handeln um Phenyl-Aminosäuren, bevorzugt Phenylalanin und Tyrosin, bei den heterocyclischen Aminosäuren handelt es sich bevorzugt um Prolin, Hydroxyprolin und Tryptophan. Besonders bevorzugt sind jedoch aliphatische C₂- C₆-Monoamino-monocarbonsäuren, worin die Aminogruppe substituiert ist, wie oben angegeben, ganz besonders bevorzugt jedoch substituiert ist mit der Acetyl- oder Benzoylgruppe.

Bei der Alkaloidkomponente der erfindungsgemäßen Morphin-Alkaloid-Säureadditionssalze handelt es sich vorzugsweise um die Morphin-Alkaloide Morphin, Codein, Heroin, Ethylmorphin, Levorphanol oder Hydromorphon.

Allgemein sind unter den genannten erfindungsgemäßen Säureadditionssalzen solche besonders bevorzugt, deren Molmasse (MG) unterhalb von 800, bevorzugt unterhalb von 600 liegt, am besten zwischen 400 und 600.

Erfindungsgemäß werden auch Mischungen der vorstehend genannten Substanzen bereitgestellt, wobei also entweder das gleiche Morphin-Alkaloid mit verschiedenen Säurekomponenten umgesetzt ist oder die gleiche Säurekomponente mit verschiedenen Morphin-Alkaloiden kombiniert ist. Natürlich kann eine derartige Zusammensetzung auch eine Kombination dieser beiden vorstehend erwähnten Varianten enthalten. In einer bevorzugten Ausführungsform handelt es sich bei der Zusammensetzung um eine Lösung oder Suspension der erfindungsgemäßen Säureadditionssalze in Glycerin, Ethylenglykol, Ölsäure, Dimethylisosorbid und/oder Dimethylsulfoxid, wobei eine derartige Lösung oder Suspension auch noch weitere Bestandteile, wie etwa Penetrationsverstärker, enthalten kann.

Unter den. Penetrationsverstärkern sind Polyoxyethylensorbitanfettsäureester wie etwa Tween 20 oder Polyoxyethylenalkohole wie z. B. Polymerisationsprodukte von bis zu 10 Molekülen Ethylenoxid mit je einem Molekül Octanol, Decanol oder Dodecanol oder Mischungen dieser Polymerisationsprodukte besonders bevorzugt.

Die erfindungsgemäßen Morphin-Alkaloid-Säureadditionssalze werden mittels bekannter Verfahrensschritte hergestellt. Ein solches Herstellungsverfahren umfaßt die Schritte, daß eine Lösung des basischen Alkaloids vorgelegt, in einem weiteren Schritt mit äquimolaren Mengen einer Lösung der organischen Säure oder - falls die Säure flüssig ist - direkt mit dieser umgesetzt und das so erhaltene Additionssalz mittels üblicher Verfahrensschritte isoliert wird.

Erfindungsgemäß werden die vorstehend beschriebenen Substanzen bzw. Stoffe oder Zusammensetzungen in Mitteln zur transdermalen oder transmucosalen Verabreichung eingesetzt. Sie werden insbesondere zur Schmerzbekämpfung oder bei der Entzugstherapie von Drogenabhängigen verwendet. Bei derartigen Mitteln zur transdermalen oder transmucosalen Verabreichung handelt es sich beispielsweise um eine Lotion, eine Salbe, Creme, ein Gel oder Spray, ein transmucosales therapeutisches System, ein transdermales therapeutisches System (TTS) oder eine iontophoretische Vorrichtung. Derartige transdermale oder transmucosale therapeutische Systeme sind dem Fachmann grundsätzlich bekannt. Beispielsweise sind sie in "Therapeutische Systeme" [Klaus Heilmann, 4. Aufl. Ferdinand Enke Verlag, Stuttgart (1984)] beschrieben.

Handelt es sich bei dem Mittel zur transdermalen Verabreichung um ein TTS, so umfaßt dieses eine - bevorzugt wirkstoffundurchlässige - Rückschicht und eine Reservoirschicht. Die Reservoirschicht enthält bevorzugt 40 - 80 Gew.-% Polymermaterial. Dieses Polymermaterial ist bevorzugterweise ausgewählt aus der Gruppe der Polyacrylate, Silikone oder Polystyrole. Des weiteren enthält die Reservoirschicht vorzugsweise 0,1 - 30 Gew.-% Weichmacher sowie die erfindungsgemäßen Morphin-Alkaloid-Säureadditionssalze in einer Menge von 0,1 bis 30 Gew.-%.

Die Rückschicht kann aus flexiblen oder nicht flexiblen Material bestehen. Materialien, die zu ihrer Herstellung verwendet werden, sind beispielsweise Polymerfolien oder Metallfolien wie Aluminiumfolie, die allein oder in mit einem polymeren Substrat beschichteter Form, eingesetzt werden. Es können auch textile Flächengebilde verwendet werden, sofern die Bestandteile des Reservoirs aufgrund ihrer physikalischen Beschaffenheit nicht durch sie hindurchtreten können.

Bei einer bevorzugten Ausführungsform ist die Rückschicht ein Verbundstoff aus einer mit Aluminium bedampften Schicht.

Die Reservoirschicht enthält - wie vorstehend bereits erwähnt - eine Polymermatrix und den Wirkstoff, wobei die Polymermatrix den Zusammenhalt des Systems gewährleistet. Sie umfaßt ein Grundpolymer und gegebenenfalls weitere übliche Zusätze. Die Auswahl des Grundpolymeren richtet sich nach den chemischen und physikalischen Eigenschaften der erfindungsgemäßen Salze. Beispielhafte Polymere sind Kautschuk, kautschukähnliche synthetische Homo, Co- oder Blockpolymere, Polyacrylsäureester und deren Copolymere, Polyurethane und Silikone. Grundsätzlich kommen alle Polymere in Frage, die auch bei der Herstellung von Haftklebern eingesetzt werden können und physiologisch unbedenklich sind. Besonders bevorzugt sind solche auf der Basis von Blockpolymeren von Styrol und 1,3-Dienen, Polyisobutylenen, Silikonen, Polymeren auf Acrylat- und/- oder Methacrylatbasis.

Die Art der üblichen Zusätze hängt vom eingesetzten Polymer ab: Nach ihrer Funktion lassen sie sich einteilen in beispielsweise klebrigmachende Agenzien, Stabilisatoren, Trägerstoffe und Füllstoffe. Die hierfür in Frage kommenden physiologisch unbedenklichen Substanzen sind dem Fachmann bekannt.

Die Reservoirschicht besitzt eine solche Eigenklebrigkeit, daß ein dauernder Kontakt zur Haut sichergestellt ist. Sie kann auch mehrschichtig aufgebaut sein.

Die Wahl des Weichmachers, der gleichzeitig als Lösungsmittel dienen kann, richtet sich nach dem Wirkstoff im Polymer.

Eine ablösbare Schutzschicht, die mit der Reservoirschicht in Berührung steht und vor der Anwendung entfernt wird, kann auch aus den gleichen Materialien, wie sie zur Herstellung der Rückschicht benutzt werden, bestehen; Voraussetzung ist, daß diese Materialien ablösbar gestaltet sind wie z. B. mittels einer Silikonbehandlung. Andere ablösbare Schutzschichten sind z. B. Polytetrafluorethylen, behandeltes Papier, Cellophan, Polyvinylchlorid u. ä..

Ein TTS wird in der Regel zunächst in einer Vorstufe als Laminat vorliegen. Wird das Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

Im Falle transmucosaler Verabreichung der erfindungsgemäßen Salze ist ein mucoadhäsiver Zusatz für die schnellere Resorption durch die Schleimhaut bevorzugt.

Solche Zusätze sind beispielsweise Polyacrylsäurecarboxymethylcellulose und andere derivatisierte Polysaccharide, insbesondere Acetylstärke oder Hydroxyethylstärke oder deren Kombinationen.

Das transdermale System kann hergestellt werden, indem der Wirkstoff zusammen mit den übrigen Bestandteilen der haftklebenden Reservoirschicht, gegebenenfalls in Lösung, homogen vermischt und auf die gegebenenfalls wirkstoffundurchlässige Rückschicht aufgetragen wird, woraufhin das/die Lösemittel entfernt wird bzw. werden. Anschließend wird die Klebeschicht mit einer entsprechenden Schutzschicht versehen.

Die Erfindung wird durch die folgenden Figuren und Beispiele näher erläutert:

Es zeigen die Fig.:

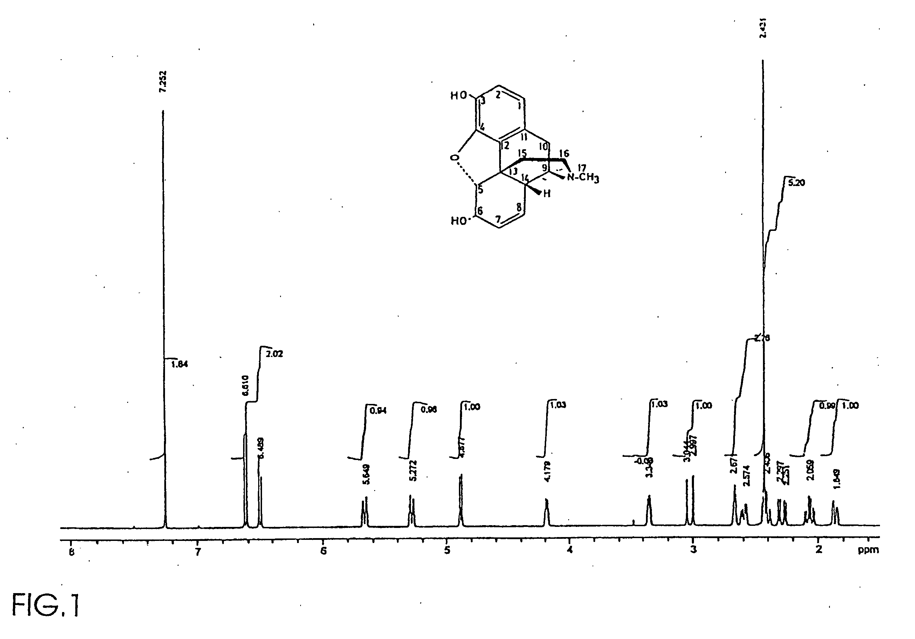

Fig. 1 zeigt das ¹H-N MR - Spektrum der Morphinbase in CDCI₃ bei 400 MHz.

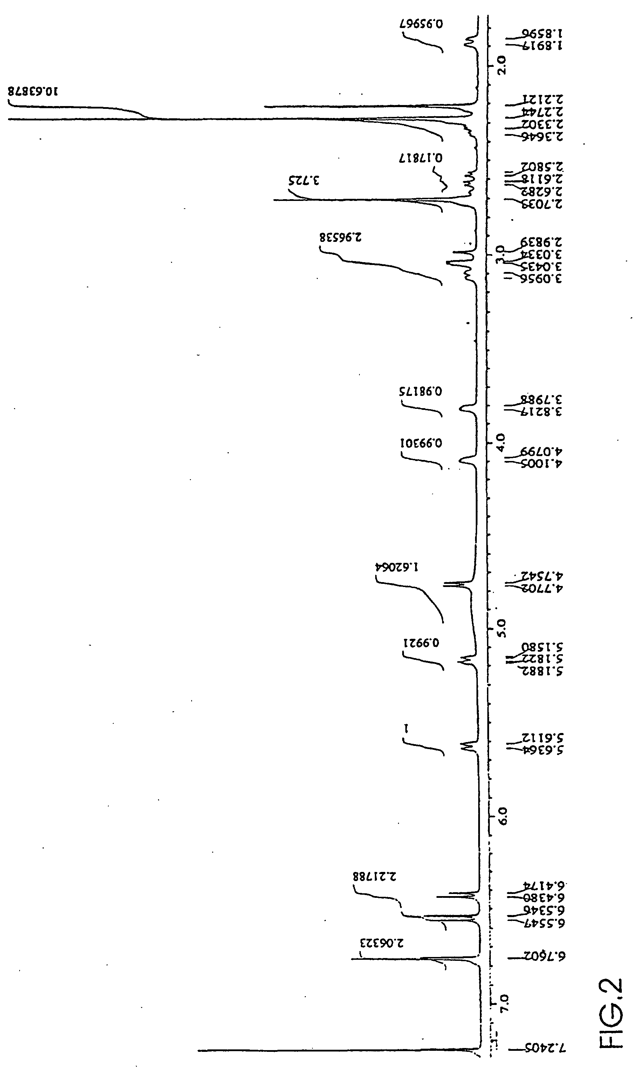

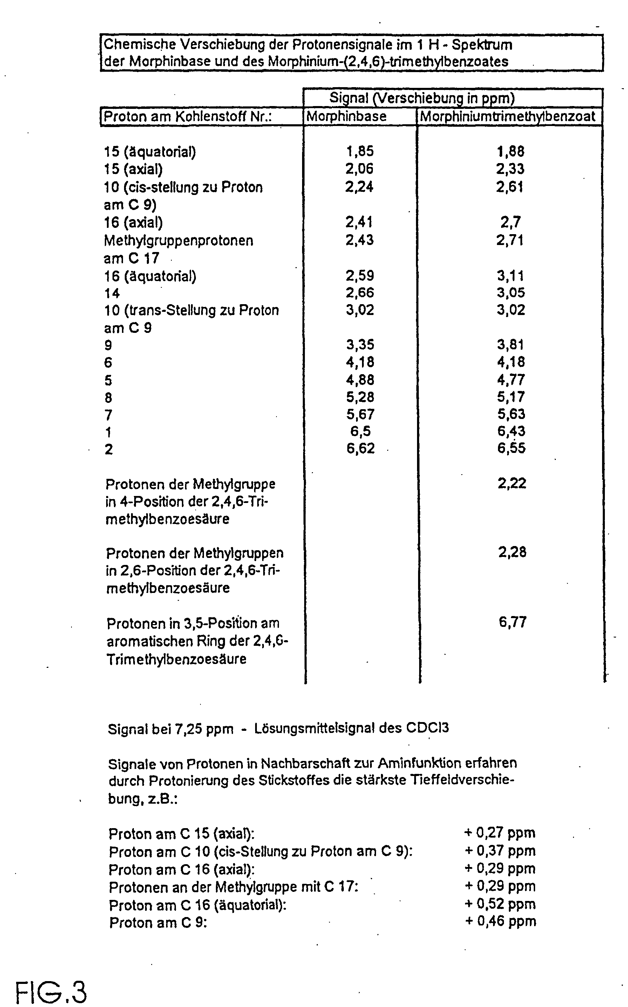

Fig. 2 zeigt das ¹H - NMR - Spektrum des Morphiniumtrimethylbenzoats in CDCI₃ bei 400 MHz.
Fig. 3:
   Die Tabelle zeigt die Zuordnung der einzelnen Protonensignale im ¹H - NMR-Spektrum der Morphin-Base sowie des Morphiniumtrimethylbenzoats entsprechend ihrer chemischen Verschiebung (Charakterisierung als Morphinium-Salz).
   Mit Hilfe der NMR-Spektroskopie läßt sich die Protonierung der Alkaloidfunktion im Morphinmolekül beobachten. Aufgrund der Salzbildung kommt es zu einer Beeinflussung der Elektronenverteilung im Piperidinteil. Eine Tieffeldverschiebung der Protonenresonanzsignale im Bereich der Basenfunktion zeigt, daß die dortigen Protonen durch Salzbildung entschirmt werden. Dies ist zum einen auf die Bindung des aciden Trimethyl-benzoesäureprotons durch das freie Elektronenpaar am basischen Stickstoff zurückzuführen; zum anderen durch den Einfluß des Trimethylbenzoesäurerestes.
Fig. 4:
   Die Tabelle zeigt die Ergebnisse von Messungen des Penetrationsverhaltens verschiedener erfindungsgemäßer Morphinsalze sowie der Vergleichssubstanzen. Die Zubereitungen wurden selbst hergestellt; die Identifizierung erfolgte über IR-ATR- und H-NMR-Spektren.
Fig. 5:
   Diese Grafik zeigt das Permeationsverhalten von Morphiniummonomethylsebacat im Vergleich zu Morphin-Base jeweils aus einem TTS, wie im Anwendungsbeispiel 1 beschrieben.
   Die Penetrationsrate des Salzes liegt ca. um den Faktor 1,8 höher als die der Base.
   Die eingearbeitete Menge des Salzes entspricht 10 Gew.-% Morphin-Base, ist also dem Referenz-TTS der Morphin-Base äquimolar.

### Herstellungsbeispiel 1:

1 g (3,5 mMol) wasserfreie Morphinbase wurden unter Erwärmen in 100 ml Methanol gelöst. Nachdem sich die Base vollständig in Methanol gelöst hatte, wurde eine Lösung von 756 mg (3,5 mMol) p-Hydroxybenzoesäure in 20 ml Methanol hinzugefügt. Die vereinigten Lösungen wurden am Rotationsverdampfer eingeengt. Nach ca. 48 h bei 5 °C war das Morphinium-p-hydroxybenzoat auskristallisiert. Lösungsmittelreste wurden mittels einer Vakuumpumpe enfernt.

### Herstellungsbeispiel 2

Herstellungsbeispiel 1 wurde wiederholt, mit der Ausnahme, daß statt der p-Hydroxybenzoesäure eine äquimolare Menge Oxoprolinsäure eingesetzt wurde.

### Herstellungsbeispiel 3

Herstellungsbeispiel 1 wurde wiederholt, mit der Ausnahme, daß statt der p-Hydroxybenzoesäure eine äquimolare Menge Hexansulfonsäure eingesetzt wurde.

### Herstellungsbeispiel 4

Herstellungsbeispiel 1 wurde wiederholt, mit der Ausnahme, daß statt der p-Hydroxybenzoesäure eine äquimolare Menge Nicotinsäure eingesetzt wurde.

### Herstellungsbeispiel 5

Herstellungsbeispiel 1 wurde wiederholt, mit der Ausnahme, daß statt der p-Hydroxybenzoesäure eine äquimolare Menge p-Aminobenzoesäure eingesetzt wurde.

### Herstellungsbeispiel 6

Herstellungsbeispiel 1 wurde wiederholt, mit der Ausnahme, daß statt der p-Hydroxybenzoesäure eine äquimolare Menge 2,4,6-Trimethylbenzoesäure eingesetzt wurde.

### Herstellungsbeisplel 7

Herstellungsbeispiel 1 wurde wiederholt, mit der Ausnahme, daß statt der p-Hydroxybenzoesäure eine äquimolare Menge Acetylglycin eingesetzt wurde.

### Herstellungsbeispiel 8

Herstellungsbeispiel 1 wurde wiederholt, mit der Ausnahme, daß statt der p-Hydroxybenzoesäure eine äquimolare Menge Hippursäure verwendet wurde.

### Vergleichsbeispiel 1

Herstellungsbeispiel 1 wurde wiederholt, mit der Ausnahme, daß statt des Salzes aus p-Hydroxybenzoesäure und Morphin lediglich eine äquimolare Menge Morphin-Base eingesetzt wurde.

### Vergleichsbeispiel 2

Herstellungsbeispiel 1 wurde wiederholt, mit der Ausnahme, daß statt der p-Hydroxybenzoesäure eine äquimolare Menge Propionsäure eingesetzt wurde.

### Vergleichsbeispiel 3

Herstellungsbeispiel 1 wurde wiederholt, mit der Ausnahme, daß statt der p-Hydroxybenzoesäure eine äquimolare Menge Ameisensäure eingesetzt wurde.

### Anwendungsbeispiel 1

1,654 g Morphiniummonomethylsebacat (entspricht 10 Gew.-% Morphin-Base) wurden in 2,346 g Ölsäure eingetragen, anschließend wurde bis zum vollständigen Auflösen des Feststoffes (ca. 15 min, visuelle Kontrolle) gerührt. Diese Lösung wurde dann wiederum unter Rühren portionsweise in 12,3 g eines selbstvernetzenden Acrylatpolymeren aus 2-Ethylhexylacrylat, Vinylacetat und Acrylsäure (48,8 Gew.-%ig, in einem Lösungsmittelgemisch Ethylacetat: Heptan : Ethanol : 2-Propanol 39 : 13 : 22 : 26) eingerührt. Anschließend wurde ca. 2 h bei Raumtemperatur gerührt. Der Verdunstungsverlust wurde mit Ethylacetat ausgeglichen. Es resultierten 10 g 48,8 Gew.-%ige wirkstoffhaltige Kleberlösung, die mit einer 350 µm Rakel auf eine aluminisierte und silikonisierte Polyethylenfolie gestrichen wurden. Nachdem die Lösemittel durch 30minütiges Trocknen bis 50°C entfernt worden waren, wurde der Kleberfilm mit einer 15 µm dicken Polyesterfolie abgedeckt. Mit geeigneten Schneidewerkzeugen wurden die vorgesehenen Applikationsflächen ausgestanzt sowie die Ränder durch Abgittern entfernt.

### Anwendungsbeispiel 2

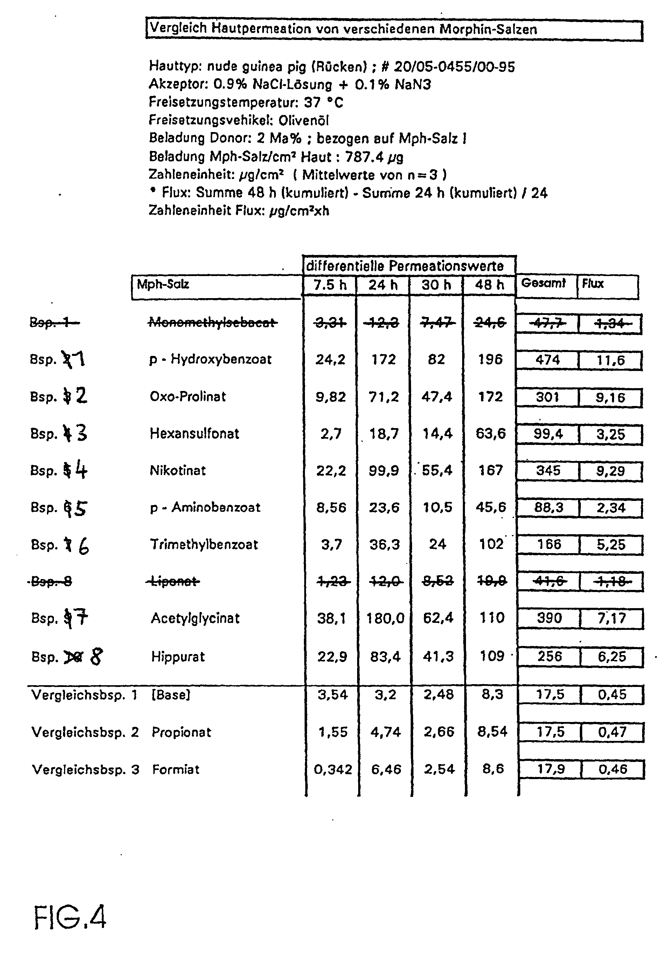

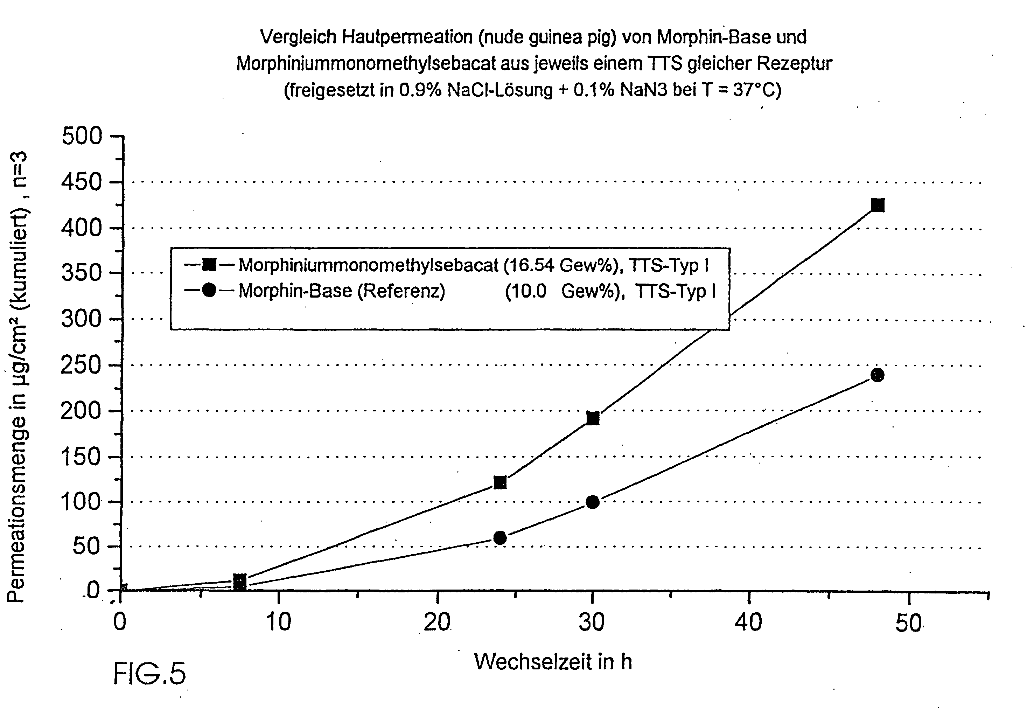

30 mg Morphinium-p-Hydroxybenzoat wurden in 1,47 g Olivenöl suspendiert. Die so erhaltene 2 Gew.-%ige Verreibung wurde mit Hilfe einer Applikationsvorrichtung auf exidierte nude guinea pig-Haut gebracht, die wiederum in einer bei 37 °C temperierten FRANZ-DiffusionszelIe eingespannt war. Als Akzeptorlösung diente 0,9-%ige Kochsalzlösung, die unter ständigem Rühren ebenfalls auf 37 °C gehalten und nach einem vorgegebenen Wechselregime komplett durch neue Akzeptorlösung ausgetauscht wurde. Die Ergebnisse der aus dem Donorteil penetrierten Mengen, durch HPLC ermittelt, sind in Fig. 4 dargestellt.

### Anwendungsbeispiele 3 bis 14

Das Anwendungsbeispiel 2 wurde mit der Abänderung wiederholt, daß statt des Morphinium-p-Hydroxybenzoates in der Verreibung die Morphiniumsalze der Herstellungsbeispiele 3 bis 10 bzw. die Stoffe der Vergleichsbeispiele 1 bis 3 eingesetzt wurden. Die Ergebnisse sind ebenfalls in der Fig. 4 dargestellt.

## Patentansprüche

1. Säureadditionssalze von Morphin-Alkaloid und organischer Säure, wobei das Morphin-Alkaloid die nachfolgende Formel I aufweist: 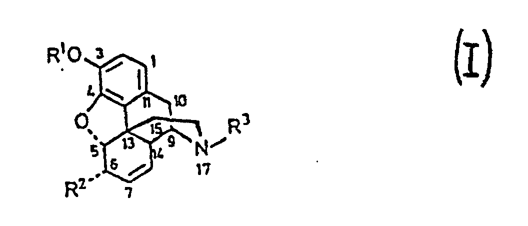 wobei R¹ ausgewählt ist aus der Gruppe, bestehend aus H, C₁- bis C₆-Alkylresten, bevorzugt Methyl-, Ethyl-, Propyl-, 1-Propyl, C(O)CH₃, R² ausgewählt ist aus der Gruppe, bestehend aus den einwertigen Resten H, OH, OC(O)CH₃, wobei in diesem Falle die vierte Valenz des (6)-C-Atoms durch H belegt ist, oder den zweiwertigen Resten =O, =CH₂, R³ ausgewählt ist aus der Gruppe, bestehend aus -CH₃, Cyclopropyl-, Cyclobutyl- und Allyl- sowie
- die Bindung an C7/C8 gesättigt sein oder am N₁₇ eine Nitroxylgruppe vorliegen kann,
**dadurch gekennzeichnet, daß** die organische Säure ausgewählt ist aus:
- Monoestern von C₃- bis C₁₈-Dicarbonsäuren mit einwertigen C₁- bis C₄-Alkoholen, insbesondere Methanol,
- C₂- bis C₆- oder C₈- bis C₁₆-Sulfonsäuren,
- der Gruppe der halogen-, *p*- und *m*-hydroxy-, alkyl-, hydroxyalkyl-, alkoxyalkyl-, und/oder alkoxysubstituierten Benzoesäuren sowie der wahlweise am N-Atom alkylierten aminosubstituierten Benzoesäuren,
- substituierten oder nicht substituierten 5- oder 6-Ringheterocyclen mit mindestens einem N-Atom oder S-Atom und mit einer Carboxylgruppenfunktion, insbesondere einer Carboxy-, Carboxymethyl-, Carboxyethyl- oder den wahlweise verzweigten Carboxypropyl- oder Carboxybutylgruppen als Substituenten,
- gesättigten oder ungesättigten, wahlweise substituierten Oxo-Carbonsäureen mit 5 bis 10 C-Atomen,
- phenoxysubstituierten gesättigten C₂- bis C₄-Carbonsäuren,
- aliphatischen, aromatischen oder heterocyclisches C₂-C₁₂-Aminosäuren, worin eine Aminogruppe substituiert ist mit einer wahlweise substituierten C₂-C₆-Alkanoylgruppe oder einer wahlweise substituierten Benzoylgruppe, und
- dass das Säureadditionssalz eine Hautpermeabilität mit einem Flux von mindestens 2,34 µg/cm²·h besitzt.

2. Säureadditionssalze von Morphin-Alkaloid und organischer Säure nach Anspruch 1, **dadurch gekennzeichnet, daß** die organische Säure ausgewählt ist aus aliphatischen Monoamino-monocarbonsäuren, worin die Aminogruppe substituiert ist mit einer C₂-C₆-Alkanoylgruppe, welche einfach oder mehrfach substituiert sein kann mit Hydroxy, C₁-C₄-Alkoxy- oder C₁-C₄-Hydroxyalkyl, oder worin die Aminogruppe substituiert ist mit dem Benzoylrest, welcher einfach oder mehrfach substituiert sein kann mit C₁-C₄-Alkyl, C₁-C₄- Alkoxy, C₁-C₄-Hydroayalkyl, Halogen, Amino oder Hydroxy.

3. Säureadditionssalze von Morphin-Alkaloid und organischer Säure nach Anspruch 2, **dadurch gekennzeichnet, daß** die organische Säure ausgewählt ist aus aliphatischen C₂-C₆-Monoamino-monocarbonsäuren, worin die Aminogruppe substituiert ist mit der Acetyl- oder der Benzoylgruppe.

4. Säureadditionssalze von Morphin-Alkaloid und organischer Säure nach Anspruch 1, **dadurch gekennzeichnet, daß** die organische Säure ausgewählt ist aus:
- hydroxy- (C₁-bis C₄)-alkyl, C₁-bis C₆-alkoxy- (C₁- bis C₄)-alkyl- oder p- oder m-hydroaysubstituierten Benzoesäuren,
Monoestern von C₅- bis C₁₀-Dicarbonsäuren, insbesondere Suberinsäure, Azelainsäure und Sebacinsäure,
C₄- bis C₈-Sulfonsäuren, insbesondere Hexansulfonsäure.

5. Säureadditionssalze von Morphin-Alkaloid und organischer Säure nach Anspruch 1, **dadurch gekennzeichnet. daß** die Säure ausgewählt ist aus C₁- bis C₄-alkylsubstituierten Benzoesäuren, bevorzugt C₁- bis C₄-trialkyl-substituierten Benzoesäuren.

6. Säureadditionssalze von Morphin-Alkaloid und organischer Säure nach Anspruch 1, **dadurch gekennzeichnet, daß** die organische Säure Hexansulfonsäure, Aminobenzoesäure oder Trimethylbenzoesäure ist.

7. Säureadditionssalze von Morphin-Alkaloid und organischer Säure nach Anspruch 1, **dadurch gekennzeichnet, daß** der 5- oder 6-Ringheterocyclus eine Pyridincarbonsäure, bevorzugt Nicotinsäure oder Liponsäure, ist.

8. Säureadditionssalze von Morphin-Alkaloid und organischer Säure nach Anspruch 1, **dadurch gekennzeichnet, daß** es sich bei der Oxo-Carbonsäure um eine wahlweise ungesättigte 2-, 4-, 5- oder 9-Oxo-Carbonsäure handelt.

9. Säureadditionssalze von Morphin-Alkaloid und organischer Säure nach Anspruch 8, **dadurch gekennzeichnet. daß** die Oxo-Carbonsäure 5-Oxopyrrolidin-2-carbonsäure, Lävulinsäure oder Oxo-dec-2-ensäure ist.

10. Säureadditionssalze von Morphin-Alkaloid und organischer Säure nach Anspruch 3, **dadurch gekennzeichnet, daß** die organische Säure Acetylglycin oder Hippursäure ist.

11. Säureadditionssalze von Morphin-Alkaloid und organischer Säure nach einem der vorhergehenden Ansprüche, **dadurch gekennzeichnet, daß** das Morphin-Alkaloid Morphin, Codein, Heroin, Ethylmorphin, Levorphanol oder Hydromorphon ist.

12. Transdermales oder transmucosales Mittel zur Verabreichung eines Säureadditionssalzes aus Morphin-Alkaloid und organischer Säure nach Anspruch 1, **dadurch gekennzeichnet, daß** es eine Lösung oder Suspension des Säureadditionssalzes in Glycerin, Ethylenglycol, Dimethylisosorbid, Ölsäure und/oder Dimethylsulfoxid umfaßt.

13. Verfahren zur Herstellung von Säureadditionssalzen nach Anspruch 1, umfassend die Schritte, daß eine Lösung des Morphin-Alkaloids vorgelegt, in einem weiteren Schritt mit äquimolaren Mengen einer Lösung der organischen Säure umgesetzt und das erhaltene Additionssalz isoliert wird.

14. Verwendung eines Säureadditionssalzes von Morphin-Alkaloid und organischer Säure nach Anspruch 1 zur Formulierung von Präparaten zur Schmerzbekämpfung oder für die Entzugstherapie von Drogenabhängigen.

15. Transdermales oder transmucosales Mittel zur Verabreichung eines Säureadditionssalzes aus Morphin-Alkaloid und organischer Säure nach Anspruch 1, **dadurch gekennzeichnet, daß** es sich um eine Lotion, Salbe, Creme, ein Gel oder Spray, eine iontophoretische Vorrichtung, ein transmucosales therapeutisches System oder ein transdermales therapeutisches System (TTS), umfassend eine wahlweise wirkstoffundurchlässige Rückschicht und eine Reservoir-schiebt, handelt.

## Claims

1. Acid addition salts of morphine alkaloid and organic acid, said morphine alkaloid having the following Formula I: 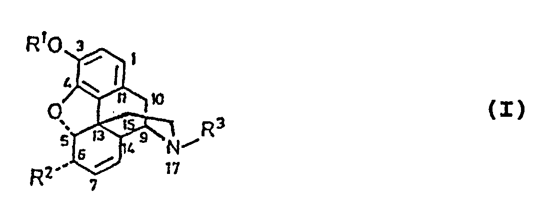 where R¹ is selected from the group consisting of H, C₁- to C₆-alkyl residues, preferably methyl, ethyl-, propyl, i-propyl, C(O)CH₃; R² is selected from the group consisting of the monad residues H, OH, OC(O)CH₃, whereby in this case the fourth valence of the (6)-C atom is occupied by H, or the dyad residues =O, =CH₂; R³ is selected from the group consisting of -CH₃, cyclopropyl, cyclobutyl and allyl; and where
- the bond at C7/C8 may be saturated, or a nitroxyl group may be present at N₁₇,
**characterized in that** the said organic acid is selected from
- monoesters of C₃- to C₁₆-dicarboxylic acids with monohydric C₁- to C₄-alcohols, especially methanol,
- C₂- to C₆- or C₈- to C₁₆- sulfonic acids,
- the group of halogen, p- and m-hydroxy, alkyl, hydroxyalkyl, alkoxyalkyl and/or alkoxy-substituted benzoic acids, as well as of the aminosubstituted benzoic acids, which may optionally be alkylated at the N atom,
- substituted or non-substituted 5-ring or 6-ring heterocycles comprising at least one N or S atom and having a carboxyl group function, especially a carboxy, carboxymethyl, carboxyethyl or the - optionally branched - carboxypropyl or carboxybutyl groups as substituents,
- saturated or unsaturated, optionally substituted, oxo-carboxylic acids having 5 to 10 C atoms,
- phenoxy-substituted saturated C₂- to C₄-carboxylic acids,
- aliphatic, aromatic or heterocylic C₂- to C₁₂-amino acids, wherein one amino group is substituted with an - optionally substituted - C₂- to C₆-alkanoyl group or an - optionally substituted - benzoyl group, and
- **in that** the acid addition salt has a skin permeability with a flux of at least 2.34 µg/cm²·h.

2. Acid addition salts of morphine alkaloid and organic acid according to Claim 1, **characterized in that** the organic acid is selected from aliphatic monoaminomonocarboxylic acids, wherein the amino group is substituted with a C₂- to C₆-alkanoyl group, which may be mono- or polysubstituted with hydroxy, C₁- to C₄-alkoxy- or C₁- to C₄-hydroxyalkyi, or wherein the amino group is substituted with the benzoyl residue, which may be mono- or polysubstituted with C₁- to C₄-alkyl, C₁- to C₄-alkoxy, C₁- to C₄-hydroxyalkyl, halogen, amino or hydroxy.

3. Acid addition salts of morphine alkaloid and organic acid according to Claim 2, **characterized in that** the organic acid is selected from aliphatic C₂- to C₆-monoaminomonocarboxylic acids, wherein the amino group is substituted with the acetyl group or the benzoyl group.

4. Acid addition salts of morphine alkaloid and organic acid according to Claim 1, **characterized in that** the organic acid is selected from:
- hydroxy-(C₁- to C₄) -alkyl, C₁- to C₆- alkoxy- (C₁- to C₄)-alkyl-substituted or p- or m-hydroxy-substituted benzoic acids,
monoesters of C₅- to C₁₀-dicarboxylic acids, especially suberic acid, azelaic acid and sebacic acid,
C₄- to C₈-sulfonic acids, especially hexanesulfonic acid.

5. Acid addition salts of morphine alkaloid and organic acid according to Claim 1, **characterized in that** the acid is selected from C₁- to C₄-alkyl-substituted benzoic acids, preferably C₁- to C₄-trialkyl-substituted benzoic acids.

6. Acid addition salts of morphine alkaloid and organic acid according to Claim 1, **characterized in that** the organic acid is hexanesulfonic acid, aminobenzoic acid or trimethylbenzoic acid.

7. Acid addition salts of morphine alkaloid and organic acid according to Claim 1, **characterized in that** the 5-ring or 6-ring heterocycle is a pyridine-carboxylic acid, preferably nicotinic acid or lipoic acid.

8. Acid addition salts of morphine alkaloid and organic acid according to Claim 1, **characterized in that** the oxocarboxylic acid is a 2-, 4-, 5- or 9-oxocarboxylic acid which is optionally unsaturated.

9. Acid addition salts of morphine alkaloid and organic acid according to Claim 8, **characterized in that** the oxocarboxylic acid is 5-oxopyrrolidine-2-carboxylic acid, levulic acid or oxodec-2-ene acid.

10. Acid addition salts of morphine alkaloid and organic acid according to Claim 3, **characterized in that** the organic acid is acetylglycin or hippuric acid.

11. Acid addition salts of morphine alkaloid and organic acid according to any one of the preceding Claims, **characterized in that** the morphine alkaloid is morphine, codeine, heroin, ethylmorphine, levorphanol or hydromorphone.

12. Transdermal or transmucosal composition for administering an acid addition salt of morphine alkaloid and organic acid according to Claim 1, **characterized in that** it comprises a solution or suspension of the acid addition salt in glycerin, ethylene glykol, dimethyl isosorbide, oleic acid and/or dimethyl sulfoxide.

13. Method for the production of acid addition salts according to Claim 1, comprising the steps of providing a solution of the morphine alkaloid, reacting, in a further step, said solution with equimolar amounts of a solution of the organic acid and isolating the resultant addition salt.

14. Use of an acid addition salt of morphine alkaloid and organic acid according to Claim 1 for formulating preparations for pain control, or for withdrawal therapy for drug addicts.

15. Transdermal or transmucosal composition for administering an acid addition salt of morphine alkaloid and organic acid according to Claim 1, **characterized in that** said composition is a lotion, an ointment, a creme, a gel or spray, an iontophoretic device, a transmucosal therapeutic system or a transdermal therapeutic system (TTS), comprising a backing layer, which optionally is active substance-impermeable, and a reservoir layer.

## Revendications

1. Sels d'addition d'acides d'alcaloïde de morphine et d'acide organique, dans lequel l'alcaloïde de morphine répond à la formule I ci-après : 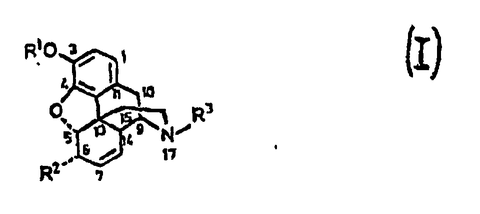 dans laquelle R¹ est choisi parmi le groupe constitué par un atome d'hydrogène, des radicaux alkyle en C₁-C₆, de préférence un radical méthyle, un radical éthyle, un radical propyle, un radical isopropyle, un groupe C(O)CH₃, R² est choisi parmi le groupe constitué par les radicaux monovalents que sont un atome d'hydrogène, un groupe OH, un groupe OC(O)CH₃, dans laquelle dans ce cas la quatrième valence de l'atome de carbone en position 6, est occupée par un atome d'hydrogène, ou constitué par les radicaux bivalents que sont un groupe =O, un groupe =CH₂, R³ est choisi parmi le groupe constitué par un groupe -CH₃, un groupe cyclopropyle, un groupe cyclobutyle et un groupe allyle, et
- la liaison sur C₇/C₈ peut être saturée, ou bien un groupe nitrosyle peut être présent sur l'atome d'azote en position 17,
**caractérisé en ce que** l'acide organique est choisi parmi le groupe comprenant :
- des monoesters d'acides dicarboxyliques en C₃-C₁₈ avec des alcools monovalents en C₁-C₄, en particulier le méthanol,
- des acides sulfoniques en C₂-C₆ ou en C₈-C₁₆,
- le groupe des acides benzoïques substitués par un atome d'halogène, par un groupe p- et m-hydroxyle, par un groupe alkyle, par un groupe hydroxyalkyle, par un groupe alcoxyalkyle et/ou par un groupe alcoxy, et des acides benzoïques substitués par un groupe amino le cas échéant alkylés sur l'atome d'azote,
- des radicaux hétérocycliques pentagonaux ou hexagonaux substitués ou non substitués comprenant au moins un atome d'azote ou un atome de soufre et une fonction de groupe carboxyle, en particulier un groupe carboxyle, un groupe carboxyméthyle, un groupe carboxyéthyle ou encore les groupes carboxypropyle ou les groupes carboxybutyle, le cas échéant ramifiés, à titre de substituants,
- des acides carboxyliques saturés ou insaturés, le cas échéant substitués par un groupe oxocarboxylique, contenant de 5 à 10 atomes de carbone,
- des acides carboxyliques saturés en C₂-C₄ substitués par un groupe phénoxy,
- des aminoacides aliphatiques, aromatiques ou hétérocycliques en C₂-C₁₂, dans lesquels un groupe amino est substitué par un groupe alcanoyle en C₂-C₆, le cas échéant substitué, ou par un groupe benzoyle, le cas échéant substitué, et
- **en ce que** le sel d'addition d'acide possède une perméabilité cutanée avec un flux d'au moins 2,34 µg/cm²·h.

2. Sels d'addition d'acides d'alcaloïde de morphine et d'acide organique selon la revendication 1, **caractérisé en ce que** l'acide organique est choisi parmi des acides monoamino-monocarboxyliques aliphatiques, dans lesquels le groupe amino est substitué par un groupe alcanoyle en C₂-C₆ qui peut être substitué une ou plusieurs fois par un groupe hydroxyle, par un groupe alcoxy en C₁-C₄ ou par un groupe hydroxyalkyle en C₁-C₆, ou dans lesquels le groupe amino est substitué par le radical benzoyle qui peut être substitué une ou plusieurs fois par un groupe alkyle en C₁-C₄, par un groupe alcoxy en C₁-C₄, par un groupe hydroxyalkyle en C₁-C₄, par un atome d'halogène, par un groupe amino ou par un groupe hydroxyle.

3. Sels d'addition d'acides d'alcaloïde de morphine et d'acide organique selon la revendication 2, **caractérisé en ce que** l'acide organique est choisi parmi des acides monoamino-monocarboxyliques aliphatiques en C₂-C₆, dans lesquels le groupe amino est substitué par le groupe acétyle ou par le groupe benzoyle.

4. Sels d'addition d'acides d'alcaloïde de morphine et d'acide organique selon la revendication 1, **caractérisé en ce que** l'acide organique est choisi parmi :
- des acides benzoïques substitués par un groupe hydroxyalkyle en C₁-C₄, par un groupe alcoxy(en C₁-C₆)alkyle en C₁-C₄ ou encore par un groupe p- ou m-hydroxyle,
- des monoesters d'acides dicarboxyliques en C₅-C₁₀, en particulier de l'acide subérique, de l'acide azélaïque et de l'acide sébacique,
- des acides sulfoniques en C₄-C₉, en particulier l'acide hexanesulfonique.

5. Sels d'addition d'acides d'alcaloïde de morphine et d'acide organique selon la revendication 1, **caractérisé en ce que** l'acide est choisi parmi des acides benzoïques substitués par un groupe alkyle en C₁-C₄, de préférence par des acides benzoïques substitués par un groupe trialkyle en C₁-C₄.

6. Sels d'addition d'acides d'alcaloïde de morphine et d'acide organique selon la revendication 1, **caractérisé en ce que** l'acide organique est l'acide hexanesulfonique, l'acide aminobenzoïque ou l'acide triméthylbenzoïque.

7. Sels d'addition d'acides d'alcaloïde de morphine et d'acide organique selon la revendication 1, **caractérisé en ce que** le radical hétérocyclique pentagonal ou hexagonal est un acide pyridinecarboxylique, de préférence l'acide nicotinique ou l'acide lipoïque.

8. Sels d'addition d'acides d'alcaloïde de morphine et d'acide organique selon la revendication 1, **caractérisé en ce que**, en ce qui concerne l'acide oxo-carboxylique, il s'agit d'un acide 2-, 4-, 5- ou 9-oxo-carboxylique, le cas échéant insaturé.

9. Sels d'addition d'acides d'alcaloïde de morphine et d'acide organique selon la revendication 8, **caractérisé en ce que** l'acide oxo-carboxylique est l'acide 5-oxo-pyrrolidine-2-carboxylique, l'acide lévulique ou l'acide oxo-déc-2-énique.

10. Sels d'addition d'acides d'alcaloïde de morphine et d'acide organique selon la revendication 3, **caractérisé en ce que** l'acide organique est l'acétylglycine ou l'acide hippurique.

11. Sels d'addition d'acides d'alcaloïde de morphine et d'acide organique selon l'une quelconque des revendications précédentes, **caractérisé en ce que** l'alcaloïde de morphine est la morphine, la codéine, l'héroïne, l'éthylmorphine, le lévorphanol ou l'hydromorphone.

12. Agent transdermique ou transmuqueux pour l'administration d'un sel d'addition d'acide d'alcaloïde de morphine et d'acide organique selon la revendication 1, **caractérisé en ce qu'**il comprend une solution ou une suspension du sel d'addition d'acide dans du glycérol, dans de l'éthylèneglycol, dans du diméthylisosorbide, dans de l'acide oléique et/ou dans du diméthylsulfoxyde.

13. Procédé pour la préparation de sels d'addition d'acides selon la revendication 1, comprenant les étapes consistant à déposer au préalable une solution de l'alcaloïde de morphine, à faire réagir cette dernière, dans une étape ultérieure, avec des quantités équimolaires d'une solution de l'acide organique, et à isoler le sel d'addition ainsi obtenu.

14. Utilisation d'un sel d'addition d'acide d'alcaloïde de morphine et d'acide organique selon la revendication 1, pour la formulation de préparations pour lutter contre la douleur ou pour la thérapie de désintoxication de toxicomanes.

15. Agent transdermique ou transmuqueux pour l'administration d'un sel d'addition d'acide d'alcaloïde de morphine et d'acide organique selon la revendication 1, **caractérisé en ce qu'**il s'agit d'une lotion, d'un onguent, d'une crème, d'un gel ou d'un spray, d'un dispositif ionothérapeutique, d'un système thérapeutique transmuqueux ou d'un système thérapeutique transdermique (TTS) comprenant une couche dorsale, le cas échéant imperméable à la substance active, et une couche faisant office de réservoir.
